# FASCICULE DE BREVET EUROPEEN

(11) **EP 3 706 902 B1**
(45) Date de publication et mention de la délivrance du brevet: **15.05.2024**
(21) Numéro de dépôt: 18822416.6
(22) Date de dépôt: 08.11.2018
(51) Int. Cl.: C07C 41/03, C07C 43/11, B01J 27/26, C08G 65/26

(54) **ALCOOL SECONDAIRE ALCOXYLÉ**
ALKOXYLIERTER SEKUNDÄRER ALKOHOL
ALKOXYLATED SECONDARY ALCOHOL

(30) Priorité: 10.11.2017 FR 1760586
(43) Date de publication de la demande: 16.09.2020
(73) Titulaire: Arkema France, 92700 Colombes (FR)
(72) Inventeur: GILLET, Jean-Philippe, 69530 Brignais (FR); BEILLON, Thierry, 69110 Sainte Foy Les Lyon (FR)
(86) Numéro de dépôt international: PCT/FR2018/052761
(87) Numéro de publication internationale: WO 2019/092366

(56) Documents cités:
- WO-A1-01/04183
- WO-A1-2009/039018
- GB-A- 758 061
- US-A- 3 395 170
- US-A- 3 803 238
- US-A1- 2010 122 622
- H.JOHNSON ET AL.,: "Topical mosquito repellents VII: alkyl triethylene glycol monoethers", J.PHARM.SCI., vol. 64, no. 4, 1 avril 1975 (1975-04-01), pages 693-695, XP002781738,
- G.J.STOCKBURGER ET AL.,: "The reactions of alkylene oxides with various butyl and other alcohols", J.AM.OIL.CHEM.SOC., vol. 40, no. 10, 1 octobre 1963 (1963-10-01), pages 590-594, XP002781739, DOI: 10.1007/BF02822474

## Description

La présente invention concerne le domaine général des alcools secondaires alcoxylés.

Les alcoxylats d'alcools secondaires représentent une famille de composés offrant un large éventail de propriétés. En effet, les applications sont multiples. Ils peuvent notamment être utilisés en tant que solvant, hydrotrope ou encore tensioactif non ionique. Ils peuvent également jouer le rôle de matière première pour d'autres composés, tels que les étheramines ou des tensioactifs anioniques obtenus par phosphatation ou sulfatation. Ainsi, les alcoxylats d'alcools secondaires constituent une classe de composés revêtant un intérêt industriel majeur pour de nombreux acteurs.

De manière classique, les alcoxylats d'alcools secondaires sont synthétisés à l'aide d'une catalyse basique, en utilisant par exemple de l'hydroxyde de potassium. Un autre type de catalyseur peut également être employé : le catalyseur de type cyanure dimétallique, appelé catalyseur DMC.

Divers documents font référence à l'alcoxylation de divers composés, dont les alcools, par une catalyse basique et/ou par une catalyse DMC. L'alcoxylation du 2-octanol est rapporté en présence de BF3 dans les documents US 3 395 170 et/ou US 3 803 238.

Par exemple, le document WO 2012/071149 décrit l'éthoxylation d'alcools secondaires polyramifiés par une catalyse basique et/ou une catalyse DMC. Dans ce document, les alcools secondaires comprennent un nombre important d'atomes de carbone et de nombreuses ramifications ou branchements sur la chaîne principale.

Par ailleurs, le document WO 2009/000852 décrit un procédé d'alcoxylation de différents composés via une catalyse DMC. De l'oxyde de propylène et/ou de l'oxyde de butylène réagissent tout d'abord avec lesdits composés, en présence d'un catalyseur DMC, puis de l'oxyde d'éthylène est greffé à l'alcoxylat synthétisé en utilisant le catalyseur DMC présent initialement.

Le document WO 2012/005897 divulgue notamment des alcools alcoxylés à l'aide d'un bloc propoxylé, puis d'un bloc éthoxylé, en présence d'un catalyseur DMC.

Il est par ailleurs connu que les alcools de faible masse moléculaire sont des poisons du catalyseur DMC.

C'est une des raisons pour lesquelles les acteurs industriels privilégient l'alcoxylation d'alcools à chaîne longue ou très fortement ramifiée pour éviter la formation de chélates stables, en présence du catalyseur DMC.

Il est également à noter que les acteurs industriels privilégient une première étape d'alcoxylation avec un bloc oxyde de propylène et/ou oxyde de butylène, puis une seconde étape d'alcoxylation à l'aide d'oxyde d'éthylène.

Les procédés d'alcoxylation décrits dans les deux documents WO 2009/000852 et WO 2012/005897 tels que cités ci-dessus sont d'ailleurs réalisés selon cette méthode.

Il est également reconnu que les alcools secondaires présentent une faible réactivité par rapport aux alcools primaires. De ce fait, l'industrialisation de produits obtenus en alcoxylant des alcools secondaires n'a jamais été raisonnablement envisagée.

En outre, à l'heure où l'enjeu environnemental est véritablement important, l'utilisation d'un réactif bio-sourcé ou biodégradable et présentant un bon profil écotoxicologique est intéressant à envisager.

Ainsi, il est recherché un alcool secondaire à chaine courte, alcoxylé, dont l'alcoxylation est réalisée par un procédé simple et permet un développement industriel et commercial à faible coût. Il serait également avantageux de développer des alcools secondaires alcoxylés, dont le composé de départ est un réactif bio-sourcé et biodégradable.

La présente invention a pour objet de proposer une solution permettant de résoudre les problèmes mentionnés ci-dessus.

L'invention a pour objet un procédé de préparation d'un composé de formule (I) suivante : 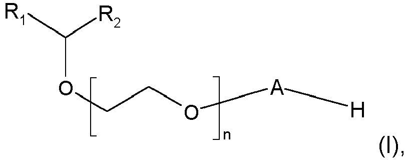 dans laquelle :
- le groupe formé par R₁, R₂ et l'atome de carbone, auquel R₁ et R₂ sont attachés, est choisi parmi le radical 2-octyle et le radical 4-méthyl-2-pentyle, plus particulièrement, le radical 2-octyle,
- n est un nombre entier compris entre, bornes incluses, 1 et 100, de préférence entre 2 et 100, de préférence encore entre 3 et 100, particulièrement entre 4 et 100, plus particulièrement entre 5 et 100, de préférence entre 6 et 100, de préférence encore entre 7 et 100, de manière préférée entre 8 et 100, encore plus préféré entre 9 et 100 et de manière très préférée entre 10 et 100,
- A représente un enchaînement d'un ou plusieurs motifs choisis parmi les motifs oxyde d'éthylène, oxyde de propylène, oxyde de butylène et leurs mélanges,
comprenant les étapes successives suivantes :
(a)faire réagir un alcool secondaire de formule (II) suivante : R1CH(OH)R2 (II), dans laquelle R1 et R2 sont tels que définis ci-dessus, avec n oxyde(s) d'éthylène, où n est tel que défini précédemment, en présence d'au moins un catalyseur de type cyanure dimétallique ;
(b)faire réagir le produit issu de l'étape (a) avec un ou plusieurs oxydes choisis parmi l'oxyde d'éthylène, l'oxyde de propylène, l'oxyde de butylène et leurs mélanges, en présence d'au moins un catalyseur de type cyanure double métallique.

Un autre objet de l'invention est l'utilisation d'un catalyseur de type cyanure dimétallique (« DiMetallic Cyanide » ou « DMC » en langue anglaise) pour réaliser l'éthoxylation du 2-octanol.

D'autres avantages et caractéristiques de l'invention apparaitront plus clairement à l'examen de la description détaillée.

Il est précisé que les expressions « de... à... » et « compris entre ... et ... » utilisées dans la présente description doivent s'entendre comme incluant chacune des bornes mentionnées.

Au sens de la présente invention, on entend par « motif oxyde d'éthylène» un motif issu de l'oxyde d'éthylène après ouverture du cycle oxirane. Au sens de la présente invention, on entend par « motif oxyde de propylène» un motif issu de l'oxyde de propylène après ouverture du cycle oxirane. Au sens de la présente invention, on entend par « motif oxyde de butylène » un motif issu de l'oxyde de butylène après ouverture du cycle oxirane.

Le composé obtenu selon le procédé de l'invention est de formule (I) telle que mentionnée ci-dessus.

Le groupe formé par R₁, R₂ et l'atome de carbone, auquel R₁ et R₂ sont attachés, est choisi parmi le radical 2-octyle et le radical 4-méthyl-2-pentyle. Plus particulièrement, le groupe formé par R₁, R₂ et l'atome de carbone, auquel R₁ et R₂ sont attachés, est le radical 2-octyle.

Avantageusement, n est compris entre, bornes incluses, 1 et 75, de préférence entre 2 et 75, de préférence encore entre 3 et 75, particulièrement entre 4 et 75, plus particulièrement entre 5 et 75, de préférence entre 6 et 75, de préférence encore entre 7 et 75, de manière préférée entre 8 et 75, encore plus préféré entre 9 et 75 et de manière très préférée entre 10 et 75.

Avantageusement, n est compris entre, bornes incluses, 1 et 50, de préférence entre 2 et 50, de préférence encore entre 3 et 50, particulièrement entre 4 et 50, plus particulièrement entre 5 et 50, de préférence entre 6 et 50, de préférence encore entre 7 et 50, de manière préférée entre 8 et 50, encore plus préféré entre 9 et 50 et de manière très préférée entre 10 et 50.

Avantageusement, n est compris entre, bornes incluses, 1 et 30, de préférence entre 2 et 30, de préférence encore entre 3 et 30, particulièrement entre 4 et 30, plus particulièrement entre 5 et 30, de préférence entre 6 et 30, de préférence encore entre 7 et 30, de manière préférée entre 8 et 30, encore plus préféré entre 9 et 30 et de manière très préférée entre 10 et 30. De préférence, n va de 2 à 30.

Le composé de formule (I) comprend n motif(s) oxyde d'éthylène, et un enchaînement comportant un ou plusieurs motifs choisis parmi le motif oxyde d'éthylène, oxyde de propylène, oxyde de butylène et leurs mélanges.

Selon un mode de réalisation particulier, lorsque le composé de formule (I) comporte un mélange desdits motifs différents, ils peuvent être répartis de manière aléatoire, alternée ou par blocs.

Dans un mode de réalisation préférée de l'invention, le composé de formule (I) comprend n motif(s) oxyde d'éthylène, et un enchaînement comportant un ou plusieurs motifs choisis parmi le motif oxyde d'éthylène, oxyde de propylène, oxyde de butylène et leurs mélanges, lesdits motifs pouvant être répartis de manière aléatoire, alternée ou par blocs, au moins un motif oxyde de propylène ou oxyde de butylène étant présent dans ledit enchaînement.

Selon un autre mode de réalisation préféré, A représente un enchaînement comprenant au moins un motif oxyde d'éthylène et au moins un motif oxyde de propylène, répartis de manière alternée, aléatoire ou par blocs.

Selon encore un autre mode de réalisation préféré, A représente un enchaînement comprenant au moins un motif oxyde d'éthylène et au moins un motif oxyde de butylène, répartis de manière alternée, aléatoire ou par blocs.

Selon encore un autre mode de réalisation préféré, A représente un enchaînement comprenant au moins un motif oxyde de propylène et au moins un motif oxyde de butylène, répartis de manière alternée, aléatoire ou par blocs.

L'invention a également pour objet un procédé de préparation d'un composé de formule (I) tel que défini précédemment, comprenant les étapes successives suivantes :
(a) faire réagir un alcool secondaire de formule (II) suivante : R₁CH(OH)R₂ (II), dans laquelle R₁ et R₂ sont tels que définis précédemment, avec n oxyde(s) d'éthylène, où n est tel que défini précédemment,
   en présence d'au moins un catalyseur de type cyanure dimétallique ;
(b) faire réagir le produit issu de l'étape (a) avec un ou plusieurs oxydes choisis parmi l'oxyde d'éthylène, l'oxyde de propylène, l'oxyde de butylène et leurs mélanges, en présence d'au moins un catalyseur de type cyanure dimétallique.

Éventuellement, le produit issu de l'étape (a) peut être isolé. Le procédé selon l'invention présente l'avantage de synthétiser le composé de formule (I) dans de bonnes conditions de sécurité, de telle sorte qu'il peut être réalisé à une échelle industrielle. En effet, les conditions opératoires en termes de température et de pression sont contrôlées grâce au procédé selon l'invention. L'exothermie de la réaction est notamment maîtrisée.

L'alcool secondaire de formule (II) est choisi parmi le 2-octanol et le méthylisobutylcarbinol, de préférence le 2-octanol. Cet alcool présente un intérêt particulier à plusieurs titres. En effet, il s'agit d'un produit bio-sourcé, biodégradable et présentant un bon profil écotoxicologique. En outre, le point d'ébullition du 2-octanol est élevé et son prix de revient est tout à fait raisonnable.

Selon un mode de réalisation préféré, l'alcool secondaire de formule (II) est mis en oeuvre après un séchage de telle sorte que la teneur en eau dans ledit alcool est inférieure ou égale à 200 ppm, de préférence inférieure ou égale à 100 ppm.

De manière préférée, le catalyseur de type cyanure dimétallique peut être de toute nature connue de l'homme du métier. Ces catalyseurs sont décrits dans les brevets US6429342, US6977236 et PL398518. Plus particulièrement, le catalyseur utilisé est l'hexacyanocobaltate de zinc, par exemple commercialisé par la Société Bayer sous le nom Arcol^{®} ou encore par la société Mexeo sous la dénomination MEO-DMC^{®}.

Avantageusement, la teneur en catalyseur de type cyanure dimétallique va de 1 à 1000 ppm par rapport à la teneur en alcool secondaire de formule (II), de préférence de 1 à 500 ppm de préférence de 2 à 300 ppm, plus préférentiellement de 5 à 200 ppm.

Selon un mode de réalisation préféré, le ratio molaire oxyde d'éthylène/ alcool secondaire de formule (II) va de 1 à 100, de préférence de 2 à 100, de préférence de 3 à 100, de préférence de 4 à 100, particulièrement de 5 à 100, plus particulièrement de 6 à 100, de manière préférée de 7 à 100, encore plus préféré de 8 à 100, préférentiellement de 9 à 100, et de préférence de 10 à 100.

De manière préférée, la température de réaction lors de l'étape (a) va de 80 à 200°C, de préférence de 100 à 180°C. La pression de la réaction lors de l'étape (a) peut aller de 0,01 MPa à 3 MPa, de préférence de 0,02 MPa à 2 MPa.

De manière préférée, la température de réaction lors de l'étape (b) va de 80 à 200°C, de préférence de 100 à 180°C. La pression de la réaction lors de l'étape (b) peut aller de 0,01 MPa à 3 MPa, de préférence de 0,02 MPa à 2 MPa.

La durée de chacune des étapes (a) et (b) peut aller de quelques minutes à quelques heures, typiquement 5 minutes à 24 heures.

De préférence, le procédé selon l'invention comprend une étape d'élimination des oxydes résiduels choisis parmi les oxydes d'éthylène, de propylène, de butylène et leurs mélanges mis en oeuvre au cours du procédé selon l'invention. Ainsi, cette étape peut se dérouler entre l'étape (a) et l'étape (b) et également après l'étape (b)

On entend au sens de la présente invention par « oxyde résiduel » un oxyde qui n'a pas réagi. De préférence, ladite étape d'élimination de l'oxyde résiduel est réalisée par cooking, c'est-à-dire par un maintien en température allant de 70 à 170°C, préférentiellement de 100 à 160°C, pour consommer l'oxyde résiduel, et/ou par une étape de stripping sous courant de gaz inerte. Alternativement, ladite étape de stripping peut être réalisée sous vide.

De préférence, après ladite étape d'élimination, la teneur massique en oxyde résiduel est inférieure ou égale à 0,1% par rapport au poids de composé de formule (I) obtenu, préférentiellement inférieure ou égale à 0,01%, plus préférentiellement inférieure ou égale à 0,001 %.

De manière préférée, le procédé selon l'invention comprend les étapes successives suivantes :
(a1) Mélanger dans un réacteur au moins un alcool secondaire de formule (II), de préférence préalablement séché comme décrit précédemment, et au moins un catalyseur de type cyanure dimétallique ;
(a2) additionner progressivement dans le mélange n oxyde(s) d'éthylène, pour obtenir l'alcool secondaire de formule (I) éthoxylé ;
(a3) maintenir à la température de réaction jusqu'à stabilisation de la pression ;
(a4) Ajouter dans le mélange un ou plusieurs oxydes choisis parmi l'oxyde d'éthylène, l'oxyde de propylène, l'oxyde de butylène et leurs mélanges,
(a5) maintenir à la température de réaction jusqu'à stabilisation de la pression ;
(a6) récupération du produit attendu après stripping éventuel (mais préféré).

De manière préférée, le procédé selon l'invention comprend les étapes successives suivantes :
(a1) Mélanger dans un réacteur au moins un alcool secondaire de formule (II), de préférence préalablement séché comme décrit précédemment, et au moins un catalyseur de type cyanure dimétallique ;
(a2) additionner progressivement dans le mélange n oxyde(s) d'éthylène, pour obtenir l'alcool secondaire de formule (I) éthoxylé ;
(a3) maintenir à la température de réaction jusqu'à stabilisation de la pression ;
(a4) ajouter dans le mélange un ou plusieurs oxydes choisis parmi l'oxyde de propylène et l'oxyde de butylène et leurs mélanges ;
(a5) maintenir à la température de réaction jusqu'à stabilisation de la pression ;
(a6) Ajouter dans le mélange un ou plusieurs oxydes choisis parmi l'oxyde d'éthylène, l'oxyde de propylène, l'oxyde de butylène et leurs mélanges ;
(a7) maintenir à la température de réaction jusqu'à stabilisation de la pression ;
(a8) récupération du produit attendu après stripping éventuel (mais préféré).

Par ailleurs, le procédé peut être mis en oeuvre en batch, en semi-continu ou en continu. L'homme du métier saura adapter le procédé de fabrication du composé de formule (I) selon l'invention selon la répartition aléatoire, alterné ou par blocs de l'enchaînement A.

L'invention a également pour objet l'utilisation d'un catalyseur de type cyanure dimétallique pour réaliser l'éthoxylation du 2-octanol.

L'invention est illustrée par les exemples suivants qui ne sont nullement limitatifs.

### EXEMPLES

Le 2-octanol (CAS RN 123-96-6) utilisé est le 2-octanol Oleris^{®} de grade « Refined » (pureté > 99%), commercialisé par Arkema France.

### Exemple 1 : Éthoxylation du 2-octanol

Dans un autoclave de 4 L, propre et sec, on charge 619 g (4,76 M) de 2-octanol séché à moins de 200 ppm d'eau et 0,06 g (100 ppm) de catalyseur DMC Arcol^{®}. Le réacteur est refermé, purgé à l'azote et l'étanchéité sous pression est vérifiée. On pressurise le réacteur à l'azote sous 0,269 MPa à 20°C.

Le milieu réactionnel est porté à 120°C sous agitation. À cette température de 120°C, on introduit 40 g d'oxyde d'éthylène. Lorsque l'amorçage de la réaction est constaté, on introduit le reste de l'oxyde d'éthylène soit en tout 628 g (14,27 M) sur 60 mn à une température de 140-150°C. En fin d'addition, on maintient la température pendant 30 mn puis on strippe à l'azote l'oxyde d'éthylène résiduel. On refroidit le réacteur à 60°C et on récupère 1240 g de 2-octanol alcoxylé comprenant 3 motifs oxyde d'éthylène. L'indice d'hydroxyle (I_{OH}) est de 210 mg de KOH/g et la coloration est de 26 Hz.

### Exemple 2 : Éthoxylation du méthylisobutylcarbinol (MIBC)

Dans un autoclave de 4 L, propre et sec, on charge 441 g (4,32 M) de MIBC séché à moins de 200 ppm d'eau et 0,044 g (100 ppm) de catalyseur DMC Arcol^{®}. Le réacteur est refermé, purgé à l'azote et l'étanchéité sous pression est vérifiée. On pressurise le réacteur à l'azote sous 0,246 MPa à 28°C.

Le milieu réactionnel est porté à 120°C sous agitation. À cette température de 120°C, on introduit 40 g d'oxyde d'éthylène. Lorsque l'amorçage de la réaction est constaté à 141°C, on introduit le reste de l'oxyde d'éthylène, soit en tout 380 g (8,64 M) sur 40 mn à une température de 140°C-150°C. En fin d'addition, on maintient la température pendant 60 mn, puis on strippe à l'azote l'oxyde d'éthylène résiduel. On refroidit le réacteur à 60°C et on récupère 815 g de méthylisobutylcarbinol alcoxylé comprenant 2 motifs oxyde d'éthylène. (I_{OH} : 290 mg de KOH/g et coloration : 3 Hz).

### Exemple 3 : Éthoxylation-propoxylation du 2-octanol

Dans un autoclave de 10 L, propre et sec, on charge 1034 g (7,95 M) de 2-octanol séché à moins de 200 ppm d'eau et 0,15 g (145 ppm) de catalyseur DMC Arcol^{®}. Le réacteur est refermé, purgé à l'azote et l'étanchéité sous pression est vérifiée. On pressurise le réacteur à l'azote sous 0,12 MPa à 27°C.

Le milieu réactionnel est porté à 120°C sous agitation. À cette température de 120°C, on introduit 35 g d'oxyde d'éthylène. Lorsque l'amorçage de la réaction est constaté, on introduit le reste de l'oxyde d'éthylène, soit en tout 2098 g (47,68 M) sur 4 heures à une température de 140°C-150°C. En fin d'addition, on maintient la température pendant 30 mn pour consommer l'oxyde d'éthylène résiduel. Un prélèvement analytique du produit intermédiaire indique les caractéristiques suivantes : I_{OH} = 136 mg de KOH/g et coloration de 39 Hz.

On poursuit la réaction en introduisant l'oxyde de propylène, soit en tout 1844 g (31,18 M) sur 3 heures. En fin de réaction, on maintient à 140°C pendant 30 mn pour consommer l'oxyde de propylène résiduel puis on procède à une purge et au dégazage, on récupère 4910 g de 2-octanol-6OE-4OP. I_{OH} = 86 mg de KOH/g et coloration de 44 Hz.

## Revendications

1. Procédé de préparation d'un composé de formule (I) suivante : 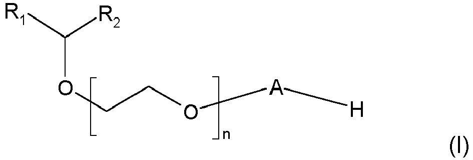 dans laquelle :
- le groupe formé par R₁, R₂ et l'atome de carbone, auquel R₁ et R₂ sont attachés, est choisi parmi le radical 2-octyle et le radical 4-méthyl-2-pentyle, plus particulièrement, le radical 2-octyle,
- n est un nombre entier compris entre, bornes incluses, 1 et 100, de préférence entre 2 et 100, de préférence encore entre 3 et 100, particulièrement entre 4 et 100, plus particulièrement entre 5 et 100, de préférence entre 6 et 100, de préférence encore entre 7 et 100, de manière préférée entre 8 et 100, encore plus préféré entre 9 et 100 et de manière très préférée entre 10 et 100,
- A représente un enchaînement d'un ou plusieurs motifs choisis parmi les motifs oxyde d'éthylène, oxyde de propylène, oxyde de butylène et leurs mélanges,
comprenant les étapes successives suivantes :
(a) faire réagir un alcool secondaire de formule (II) suivante : R₁CH(OH)R₂ (II), dans laquelle R₁ et R₂ sont tels que définis ci-dessus, avec n oxyde(s) d'éthylène, où n est tel que défini précédemment, en présence d'au moins un catalyseur de type cyanure dimétallique ;
(b) faire réagir le produit issu de l'étape (a) avec un ou plusieurs oxydes choisis parmi l'oxyde d'éthylène, l'oxyde de propylène, l'oxyde de butylène et leurs mélanges, en présence d'au moins un catalyseur de type cyanure double métallique.

2. Procédé selon la revendication 1, **caractérisé en ce que** le catalyseur de type cyanure dimétallique est le hexacyanocobaltate de zinc.

3. Procédé selon l'une quelconque des revendications précédentes, **caractérisé en ce que** la teneur en catalyseur de type cyanure dimétallique va de 1 à 1000 ppm par rapport à la teneur en alcool secondaire de formule (II), de préférence de 2 à 300 ppm, plus préférentiellement de 5 à 200 ppm.

4. Procédé selon l'une quelconque des revendications précédentes, **caractérisé en ce que** le ratio molaire oxyde d'éthylène/alcool secondaire de formule (II) va de 2 à 100, de préférence de 3 à 100, de préférence encore de 4 à 100, particulièrement de 5 à 100, plus particulièrement de 6 à 100, de manière préférée de 7 à 100, encore plus préféré de 8 à 100, préférentiellement de 9 à 100, et de préférence de 10 à 100.

5. Utilisation d'un catalyseur de type cyanure dimétallique pour réaliser l'éthoxylation du 2-octanol.

## Patentansprüche

1. Verfahren zur Herstellung einer Verbindung der folgenden Formel (I): 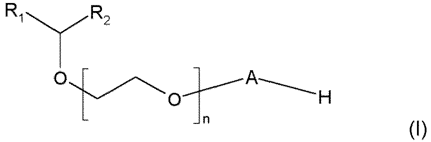 in der:
- die durch R₁, R₂ und das Kohlenstoffatom, an das R₁ und R₂ gebunden sind, gebildete Gruppe aus dem 2-Octylrest und dem 4-Methyl-2-pentylrest, insbesondere dem 2-Octylrest, ausgewählt ist,
- n für eine ganze Zahl steht, die unter Einschluss der Grenzen zwischen 1 und 100, vorzugsweise zwischen 2 und 100, weiter bevorzugt zwischen 3 und 100, speziell zwischen 4 und 100, spezieller zwischen 5 und 100, vorzugsweise zwischen 6 und 100, weiter bevorzugt zwischen 7 und 100, weiter bevorzugt zwischen 8 und 100, noch weiter bevorzugt zwischen 9 und 100 und ganz besonders bevorzugt zwischen 10 und 100 liegt,
- A für eine Sequenz einer oder mehrerer Einheiten, die aus Ethylenoxid-, Propylenoxid-, Butylenoxid-Einheiten und Mischungen davon ausgewählt sind, steht,
umfassend die folgenden aufeinanderfolgenden Schritte:
(a) Umsetzen eines sekundären Alkohols der folgenden Formel (II): R₁CH(OH)R₂ (II), in der R₁ und R₂ wie oben definiert sind, mit n Ethylenoxid(en), wobei n wie oben definiert ist, in Gegenwart mindestens eines Katalysators vom Dimetallcyanid-Typ;
(b) Umsetzen des Produkts aus Schritt (a) mit einem oder mehreren Oxiden, die aus Ethylenoxid, Propylenoxid, Butylenoxid und Mischungen davon ausgewählt werden, in Gegenwart mindestens eines Katalysators vom Doppelmetallcyanid-Typ.

2. Verfahren nach Anspruch 1, **dadurch gekennzeichnet, dass** es sich bei dem Katalysator vom Dimetallcyanid-Typ um Zinkhexacyanocobaltat handelt.

3. Verfahren nach einem der vorhergehenden Ansprüche, **dadurch gekennzeichnet, dass** der Gehalt an Katalysator vom Dimetallcyanid-Typ im Bereich von 1 bis 1000 ppm, bezogen auf den Gehalt an sekundärem Alkohol der Formel (II), vorzugsweise von 2 bis 300 ppm, weiter bevorzugt von 5 bis 200 ppm, liegt.

4. Verfahren nach einem der vorhergehenden Ansprüche, **dadurch gekennzeichnet, dass** das Molverhältnis von Ethylenoxid zu sekundärem Alkohol der Formel (II) im Bereich von 2 bis 100, vorzugsweise von 3 bis 100, weiter bevorzugt von 4 bis 100, speziell von 5 bis 100, spezieller von 6 bis 100, vorzugsweise von 7 bis 100, noch weiter bevorzugt von 8 bis 100, bevorzugt von 9 bis 100 und vorzugsweise von 10 bis 100 liegt.

5. Verwendung eines Katalysators vom Dimetallcyanid-Typ zur Durchführung der Ethoxylierung von 2-Octanol.

## Claims

1. Process for preparing a compound of formula (I) below: 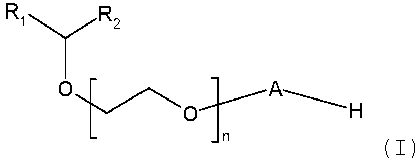 in which:
- the group formed by R₁, R₂ and the carbon atom to which R₁ and R₂ are attached is chosen from the 2-octyl radical and the 4-methyl-2-pentyl radical, more particularly the 2-octyl radical,
- n is an integer between, limits inclusive, 1 and 100, preferably between 2 and 100, more preferably between 3 and 100, particularly between 4 and 100, more particularly between 5 and 100, preferably between 6 and 100, more preferably between 7 and 100, preferably between 8 and 100, more preferably between 9 and 100 and very preferably between 10 and 100,
- A represents a sequence of one or more units chosen from ethylene oxide, propylene oxide and butylene oxide units and mixtures thereof,
comprising the following successive steps:
(a) reacting a secondary alcohol of formula (II) below: R₁CH(OH)R₂ (II), in which R₁ and R₂ are as defined above, with n ethylene oxide (s), where n is as defined as before, in the presence of at least one catalyst of dimetallic cyanide type;
(b) reacting the product obtained from step (a) with one or more oxides chosen from ethylene oxide, propylene oxide and butylene oxide and mixtures thereof, in the presence of at least one catalyst of dimetallic cyanide type.

2. Process according to Claim 1, **characterized in that** the catalyst of dimetallic cyanide type is zinc hexacyanocobaltate.

3. Process according to any one of the preceding claims, **characterized in that** the content of catalyst of dimetallic cyanide type ranges from 1 to 1000 ppm relative to the content of secondary alcohol of formula (II), preferably from 2 to 300 ppm, more preferentially from 5 to 200 ppm.

4. Process according to any one of the preceding claims, **characterized in that** the ethylene oxide/secondary alcohol of formula (II) mole ratio ranges from 2 to 100, preferably from 3 to 100, more preferably from 4 to 100, particularly from 5 to 100, more particularly from 6 to 100, preferably from 7 to 100, even more preferably from 8 to 100, preferentially from 9 to 100 and preferably from 10 to 100.

5. Use of a catalyst of dimetallic cyanide type for performing the ethoxylation of 2-octanol.
